# EUROPEAN PATENT SPECIFICATION

(11) **EP 0 674 636 B1**
(45) Date of publication and mention of the grant of the patent: **20.05.1998**
(21) Application number: 94902926.8
(22) Date of filing: 21.12.1993
(51) Int. Cl.: C07D 453/02, A61K 31/435

(54) **QUINUCLIDINE DERIVATIVES AS SQUALENE SYNTHASE INHIBITORS**
QUINUCLIDINDERIVATE ALS SQUALENE SYNTHESE INHIBITOREN
DERIVES DE LA QUINUCLIDINE UTILISES COMME INHIBITEURS DE LA SQUALENE SYNTHETASE

(30) Priority: 21.12.1992 GB 9226577
(43) Date of publication of application: 04.10.1995
(73) Proprietor: ZENECA LIMITED, London W1Y 6LN (GB)
(72) Inventor: BROWN, George Robert, Wilmslow, Cheshire SK9 6HH (GB)
(74) Representative: Bill, Kevin
(86) International application number: GB9302616
(87) International publication number: WO9414804

(56) References cited:
- WO-A-92/15579
- WO-A-93/21183

## Description

This invention concerns heterocyclic derivatives which are useful in inhibiting squalene synthase, processes for their preparation and pharmaceutical compositions containing them. The present invention is also concerned with methods of using such heterocyclic derivatives in treating diseases and medical conditions where inhibition of squalene synthase is desirable, for example in treating diseases or medical conditions such as hypercholesterolemia and atherosclerosis.

Several different classes of compounds have been reported to possess the capability of being able to lower cholesterol levels in blood plasma. For example agents which inhibit the enzyme HHG CoA reductase, which is essential for the production of cholesterol, have been reported to reduce levels of serum cholesterol. Illustrative of this class of compounds is the HMG CoA reductase inhibitor known as lovastatin which is disclosed in US Patent No 4,231,938. Other agents which are reported to lower serum cholesterol include those which act by complexing with bile acids in the intestinal system and which are hence termed "bile acid sequestrants". It is believed that many of such agents act by sequestering bile acids within the intestinal tract. This results in a lowering of the levels of bile acid circulating in the enteroheptatic system and promoting replacement of bile acids by synthesis in the liver from cholesterol, which results in an upregulation of the hepatic LDL receptor and hence in a lowering of circulating blood cholesterol levels.

Squalene synthase (also referred to in the art as squalene synthetase) is a microsomal enzyme which catalyses the first committed step of cholesterol biosynthesis. Two molecules of farnesyl pyrophosphate (FPP) are condensed in the presence of the reduced form of nicotinamide adenine dinucleotide phosphate (NADPH) to form squalene. The inhibition of this committed step to cholesterol should leave unhindered biosynthetic pathways to ubiquinone, dolichol and isopentenyl t-RNA. Elevated cholesterol levels are known to be one of the main risk factors for ischaemic cardiovacsular disease. Thus, an agent which inhibits squalene synthase should be useful in treating diseases and medical conditions in which a reduction in the levels of cholesterol is desirable, for example hypercholesterolemia and atherosclerosis.

Thus far, the design of squalene synthase inhibitors has concentrated on the preparation of analogues of the substrate farnesyl pyrophosphate (FPP), and hence on compounds which contain phosphorus groups. For example, the preparation of phosphorous-containing squalene synthase inhibitors is reported in published European Patent Application No. 409,181; and the preparation of isoprenoid (phosphinylmethyl)phosphonates as inhibitors of squalene synthase is reported by Biller et al, J. Med. Chem., 1988, 31, 1869. Recently certain quinuclidine derivatives have been reported (WO 92/15579 and US 5,135,935) to inhibit squalene synthase.

International Patent Application WO 92/15579, published on 17 September 1992, refers to polycyclic compounds containing two mono and/or bicyclic rings and a basic tertiary amino group as being inhibitors of squalene synthetase. Compounds in which the tertiary amino group is a quinuclidine moiety are disclosed.

Quinuclidine derivatives having the substituent -Ar¹-X-Ar² in which Ar¹ and Ar² are optionally substituted phenyl groups and X is a group such as -CH₂CH₂-, -CH=CH-, -C≡C-, -OCH₂-, -CH₂O- and -NHCO- are mentioned in International Patent Application WO 93/21183 (published on 28 October 1993).

The present invention is based on the discovery that certain heterocyclic derivatives are inhibitors of squalene synthase, and are hence useful in treating diseases and medical conditions in which inhibition of squalene synthase is desirable.

According to the present invention there is provided a compound of formula I (formula set out hereinafter together with the other chemical formulae referred to herein), or a pharmaceutically acceptable salt thereof, wherein:
R¹ is hydroxy;
R² is hydrogen;
X is selected from -CH₂CH₂-, -CH=CH-, -C≡C-, -CH₂O-, -OCH₂₋, -CH₂NH-, -NHCH₂₋, -CH₂CO-, -COCH₂₋, -CH=N-, -N=CH-, -O-, -NH-, -CO-, -CH₂₋, -CH₂S-, -SCH₂₋ and -S- (wherein the sulphur atom in the latter three groups may optionally bear one or two oxygen atoms);
Ar¹ is a phenylene moiety;
Ar² is a heterocyclic moiety;
and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, d- [(1 -6C)alkyl]amino N-(1-6C)alkylcarbamoyl, di-N,N-[(1-6C)alkyl]carbamoyl, (1 -6C)alkoxycarbonyl, (1-6C)alkylthio,(1 -6C)alkylsulphinyl, (1-6C)alkylsulphonyl, halogeno(1-6C)alkyl, (1 -6C)alkanoylamino,ureido, N'-(1-6C)alkylureido, (1-6C)alkanoyl and oxime derivatives thereof and O-(1-6C)alkyl ethers of said oxime derivatives.

It will be understood that when formula I compounds contain a chiral centre (or centres), the compounds of the invention may exist in, and be isolated in, optically active or racemic form. The invention includes any optically active or racemic form of a compound of formula I which possesses the beneficial pharmacological effect of inhibiting squalene synthase. The synthesis of optically active forms may be carried out by standard techniques of organic chemistry well known in the art, for example by, resolution of a racemic form, by synthesis from optically active starting materials or by asymmetric synthesis.

It will also be understood that, insofar as certain of the compounds of the formula I may exhibit the phenomenon of tautomerism, for example a compound of formula I in which Ar² bears a hydroxy substituent, the present invention includes any tautomeric form of a compound of formula I which possesses the beneficial pharmacological effect of inhibiting squalene synthase.

It will also be understood that some of the compounds of formula I may exist as geometric isomers and that in such cases the present invention will include any such isomer which possesses the beneficial pharmacological effect of inhibiting squalene synthase.

It is also to be understood that generic terms such as "alkyl" include both the straight chain and branched chain groups such as butyl and tert-butyl. However, when a specific term such as "butyl" is used, it is specific for the straight chain or "normal" butyl group, branched chain isomers such as "t-butyl" being referred to specifically when intended.

Ar², the heterocyclic moiety, encompases monocyclic aromatic heterocycles which contains (in addition to carbon atoms) one, two or three heteroatoms selected from nitrogen, oxygen and sulphur; bicyclic aromatic heterocycles of about 8 to 10 ring atoms and containing one, two or three heteroatoms selected from nitrogen, oxygen and sulphur, and in particular, benz-derivatives of said monocyclic aromatic heterocycles; as well as bicyclic heterocycles which consists of a non-aromatic 5-membered or 6-membered heterocyclic ring containing (in addition to carbon atoms) one, two or three heteroatoms selected from nitrogen, oxygen and sulphur which is fused to a benzene ring.

It will be appreciated that the heterocyclic moiety may be attached to -XAr¹ through any available ring atom. It will therefore be appreciated that the heterocyclic moiety will, in general, be attached to -XAr¹ through a ring carbon atom. In particular, it will be appreciated that for some values of X the stability of the group -Ar¹-X-Ar² means that it is generally preferred that Ar² is attached to -XAr¹ through a ring carbon atom on Ar² rather than through a ring nitrogen atom. It is generally preferred that Ar² is attached to -XAr¹ through a ring carbon atom on Ar².

Suitable values for Ar² will therefore include, for example, an aromatic 5-membered or 6-membered heterocyclic ring containing one, two or three heteroatoms selected from nitrogen, oxygen and sulphur, and an aromatic 5-membered or 6-membered heterocyclic ring containing one, two or three heteroatoms selected from nitrogen, oxygen and sulphur which is fused to a benzene ring.

Suitable values for Ar¹, the phenylene moiety, include 1,2-phenylene, 1,3-phenylene and 1,4-phenylene.

A particular value for an optional substituent which may be present on Ar¹ or Ar² is, for example,
- for alkyl;: (1-4C)alkyl, such as methyl, ethyl, propyl, isopropyl, butyl, isobutyl or sec-butyl;
- for alkenyl;: (2-4C)alkenyl, such as allyl, prop-2-enyl, but-2-enyl or 2-methyl-2-propenyl;
- for alkynyl;: (2-4C)alkynyl, such as prop-2-ynyl or but-2-ynyl;
- for alkoxy;: (1-4C)alkoxy, such as methoxy, ethoxy, propoxy, isopropoxy or butoxy;
- for alkylamino;: (1-4C)alkylamino, such as methylamino, ethylamino, propylamino or butylamino;
- for di-alkylamino;: di-[(1-4C)alkylamino, such as dimethylamino, diethylamino, methylpropylamino or dipropylamino;
- for alkylcarbamoyl;: N-methylcarbamoyl, N-ethylcarbamoyl or N-propylcarbamoyl;
- for di-alkylcarbamoyl;: N,N-dimethylcarbamoyl or N,N-diethylcarbamoyl;
- for alkoxycarbonyl;: methoxycarbonyl, ethoxycarbonyl or propoxycarbonyl;
- for alkylthio;: methylthio, ethylthio, propylthio, isopropylthio or butylthio;
- for alkylsulphinyl;: methylsulphinyl, ethylsulphinyl, propylsulphinyl, isopropylsulphinyl or butylsulphinyl;
- for alkylsulphonyl;: methylsulphonyl, ethylsulphonyl, propylsulphonyl, isoproylsulphonyl or butylsulphonyl;
- for halogeno;: fluoro, chloro, bromo or iodo;
- for halogenoalkyl;: halogenoalkyl containing one, two or three halo groups selected from fluoro, chloro, bromo and iodo and an alkyl group selected from methyl, ethyl, propyl, iso-propyl, butyl, iso-butyl and sec-butyl, (in particular fluoromethyl, difluoromethyl or trifluoromethyl);
- for alkanoyl;: formyl, acetyl, propionyl and butyryl;
- for 0-(1-6C)alkyl ethers of alkanoyl oximes: methyl, ethyl, propyl, isopropyl and butyl esters of said oximes;
- for alkylureido;: N'-methylureido, N'-ethylureido, N'-propylureido, N'-isopropylureido and N'-butylureido; and
- for alkanoylamino;: formamido, acetamido, propionamido, iso-propionamido, butyramido or iso-butyramido.

A particular value for Ar¹ is, for example, 1,3-phenylene or 1,4-phenylene moiety, especially a 1,4-phenylene moiety.

Particular values for Ar² include, for example, furyl, pyrrolyl, thienyl, pyridyl, pyrimidinyl, pyridazinyl, imidazolyl, oxazolyl, isooxazolyl, thiazolyl, isothiazolyl, oxadiazolyl, 1,2,3-triazolyl, 1,2,4-triazolyl, benzfuranyl, quinolyl, isoquinolyl, benzimidazolyl, indolyl, benzthiazolyl, benzodioxolyl (such as 1,3-benzodioxolyl), benzodioxanyl (such as 1,4-benzodioxanyl) and benzodihydrofuranyl.

In one embodiment X is, for example, -CH₂CH₂-, -CH=CH-, -C≡C-, -CH₂O-, -OCH₂-, -CH₂NH-, -NHCH₂-, -CH₂-, -CO-, -CH₂CO-, -COCH₂-, -CH₂S- or -SCH₂- (wherein the sulphur atom in the latter two groups may optionally bear one or two oxygen atoms).

In a further embodiment X is, for example, -CH₂CH₂-, -CH=CH-, -C≡C-, -CH₂O-, -OCH₂-, -CH₂NH-, -NHCH₂-, -CH₂CO-, -COCH₂-, -O-, -NH-, -CH₂S-, -SCH₂- or -S- (wherein the sulphur atom in the latter three groups may optionally bear one or two oxygen atoms).

In a further embodiment X is, for example, -0-, -CO-, -S-, -S(O)-, -S(O)₂-, -NH- or -CH₂-.

In a further embodiment X is, for example, -CH₂CH₂-, -CH=CH- or -C≡C-.

In a particular embodiment, one or both of Ar¹ and Ar² may optionally bear one or more substituents selected from halogeno, hydroxy, nitro, (1-6C)alkyl, (2-6C)alkenyl, (1-6C)alkoxy, (1-6C)alkanoyl and oxime derivatives thereof and O-(1-6C)alkyl ethers of said oximes, (1-6C)alkanoylamino and halogeno-(1-6C)alkyl.

In general, it is preferred that Ar¹ is optionally unsubstituted or substituted by one, two or three substituents independently selected from those mentioned above; and that Ar² is optionally unsubstituted or substituted by one, two or three substituents from those mentioned above.

In general it is preferred, for example, that Ar¹ is a 1,4-phenylene moiety.

In general it is preferred, for example, that Ar² is selected from a pyrrolyl, thienyl, pyridyl, quinolyl and a 1,3-benzodioxolyl moiety.

Values of Ar² of interest include, for example, furyl, pyrrolyl, thienyl, pyridyl, pyrimidinyl, pyridazinyl, imidazolyl, oxazolyl, isooxazolyl, thiazolyl, isothiazolyl, oxadiazolyl, 1,2,3-triazolyl, 1,2,4-triazolyl, benzfuranyl, quinolyl, isoquinolyl, benzimidazolyl, indolyl, benzthiazolyl, benzodioxolyl (such as 1,3-benzodioxolyl), benzodioxanyl (such as 1,4-benzodioxanyl) and benzodihydrofuranyl; which may optionally bear one or two substituents independently selected from fluoro, chloro, bromo, iodo, hydroxy, nitro, methyl, ethyl, propyl, isopropyl, butyl, isobutyl, sec-butyl, allyl, methoxy, ethoxy, propoxy, isopropoxy, butoxy, formyl, acetyl, propionyl, butyryl and oxime derivatives of the last four groups and O-methyl, ethyl, propyl, isopropyl and butyl ethers of said oximes, acetamido, propionamido, iso-propionamido, fluoromethyl, difluoromethyl and trifluoromethyl.

Values of Ar¹ of interest include, for example, an unsubstituted 1,4-phenylene moiety and a 1,4-phenylene moiety having one or two substituents independently selected from fluoro, chloro, bromo, iodo, hydroxy, nitro, methyl, ethyl, propyl, isopropyl, butyl, isobutyl, sec-butyl, allyl, methoxy, ethoxy, propoxy, isopropoxy, butoxy, formyl, acetyl, propionyl, butyryl and oxime derivatives of the last four groups and O-methyl, ethyl, propyl, isopropyl and butyl ethers of said oximes, acetamido, propionamido, iso-propionamido, fluoromethyl, difluoromethyl and trifluoromethyl.

Further values for Ar² include, for example, 2-thienyl, 2-pyridyl, 3-pyridyl, 2-pyrrolyl, 2-imidazolyl, 1,2,3-triazol-4-yl, 1,2,4-triazol-3-yl, 3-quinolyl, 2-quinolyl, 2-thiazolyl, 5-thiazolyl, 1-isoquinolyl, 3-isoquinolyl, 2-benzimidazolyl, 2-benzthiazolyl, 5-oxadiazoyl, 2-indolyl and 3-indolyl, which may optionally bear one or two substituents independently selected from fluoro, chloro, bromo, iodo, hydroxy, nitro, methyl, ethyl, propyl, isopropyl, butyl, isobutyl, sec-butyl, allyl, methoxy, ethoxy, propoxy, isopropoxy, butoxy, formyl, acetyl, propionyl, butyryl and oxime derivatives of the last four groups and O-methyl, ethyl, propyl, isopropyl and butyl ethers of said oximes, acetamido, propionamido, iso-propionamido, fluoromethyl, difluoromethyl and trifluoromethyl.

Specific values for X include, for example, -CH=CH-, CH₂O- and -O-.

Specific values for Ar¹ include, for example, a 1,4-phenylene moiety.

Specific values for Ar² include, for example, N-methylpyrrol-2-yl, 2-thienyl, 2-pyridyl, 2-quinolyl and 1,3-benzodioxol-5-yl.

In a specific embodiment Ar¹ is an unsubstituted 1,4-phenylene moiety and Ar² is an optionally substituted heterocyclic moiety -(as hereinbefore defined).

In a particular embodiment of the present invention there is provided a compound of formula I, or a pharmaceutically acceptable salt thereof wherein R¹ is hydroxy; R² is hydrogen; X is selected from -CH₂CH₂-, -CH=CH-, -C≡C-, -CH₂O-, -OCH₂-, -CH₂NH-, -NHCH₂-, -CH₂CO-, -COCH₂-, -CH=N-, -N=CH-, -O-, -NH-, -CO-, -CH₂-, -CH₂S-, -SCH₂- and -S- (wherein the sulphur atom in the latter three groups may optionally bear one or two oxygen atoms);
Ar¹ is a 1,4-phenylene moiety, Ar² is a heterocyclic moiety; and wherein one or both of Ar¹ and Ar² may optionally bear one or more substituents independently selected from halogeno, hydroxy, amino, nitro cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino, N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives.

Particular, preferred and specific values are the appropriate values mentioned above.

In a further embodiment there is provided a compound of formula I, or a pharmaceutically acceptable salt thereof, wherein:
R¹ is hydroxy;
R² is hydrogen; or
X is selected from -CH₂CH₂-, -CH=CH-, -C≡C-, -CH₂O-, -OCH₂-, -CH₂NH-, -NHCH₂-, -CH₂CO-, -COCH₂-, -CH₂S- and -SCH₂- (wherein the sulphur atom in the latter two groups may optionally bear one or two oxygen atoms); Ar¹ is a 1,4-phenylene moiety; Ar² is a heterocyclic moiety;
and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives.

Particular, preferred and specific values are the appropriate values mentioned above.

In a further embodiment there is provided a compound of formula I (formula set out hereinafter together with the other chemical formulae referred to herein), or a pharmaceutically acceptable salt thereof, wherein:
R¹ is hydroxy;
R² is hydrogen;
X is selected from -CH₂CH₂-, -CH=CH-, -C≡C-, -CH₂O-, -OCH₂-, -CH₂NH-, -NHCH₂-, -CH₂CO-, -COCH₂-, -CH₂-, -CO-, -O-, -NH-, -CH₂S-, -SCH₂- and -S- (wherein the sulphur atom in the latter three groups may optionally bear one or two oxygen atoms); Ar¹ is a 1,4-phenylene moiety; Ar² is a heterocyclic moiety;
and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives.

Particular, preferred and specific values are the appropriate values mentioned above.

In a further embodiment there is provided a compound of formula I (formula set out hereinafter together with the other chemical formulae referred to herein), or a pharmaceutically acceptable salt thereof, wherein:
R¹ is hydroxy;
R² is hydrogen;
X is selected from -0-, -S-, -NH- or -CH₂-; Ar¹ is a 1,4-phenylene moiety; Ar² is a heterocyclic moiety;
and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives.

Particular, preferred and specific values are the appropriate values mentioned above.

In a further embodiment there is provided a compound of formula I (formula set out hereinafter together with the other chemical formulae referred to herein), or a pharmaceutically acceptable salt thereof, wherein:
R¹ is hydroxy; R² is hydrogen;
X is selected from -CH₂CH₂-, -CH=CH-, -C≡C-, -CH₂O-, -OCH₂-, -CH₂NH-, -NHCH₂-, -CH₂CO-, -COCH₂-, -CH=N-, -N=CH-, -CO-, -O-, -NH-, -CH₂-, -CH₂S-, -SCH₂- and -S- (wherein the sulphur atom in the latter three groups may optionally bear one or two oxygen atoms); Ar¹ is a phenylene moiety; Ar² is a heterocyclic moiety;
and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives.

Particular, preferred and specific values are the appropriate values mentioned above.

In an embodiment of particular interest R¹ is hydroxy; R² is hydrogen; X is selected from -CH₂CH₂-, -CH=CH-, -C≡C-, -CH₂O-, -OCH₂-, -CH₂NH-, -NHCH₂-, -CH₂CO-, -COCH₂-, -CO-, -O-, -NH-, -CH₂-, -CH₂S-, -SCH₂- and -S- (wherein the sulphur atom in the latter three groups may optionally bear one or two oxygen atoms); Ar¹ is a 1,4-phenylene moiety; Ar² is a 5-membered or 6-membered heteroaryl moiety containing one, two or three heteroatoms selected from nitrogen, oxygen and sulphur; and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives.

Particular, preferred and specific values are the appropriate values mentioned above.

In a specific embodiment R¹ is hydroxy; R² is hydrogen; X is selected from -CH₂CH₂-, -CH=CH-, -C≡C-, -CH₂O-, -OCH₂-, -CH₂NH-, -NHCH₂-, -CH₂CO-, -COCH₂-, -CO-, -O-, -NH-, -CH₂-, -CH₂S-, -SCH₂- and S; Ar¹ is a 1,4-phenylene moiety; Ar² is pyridyl; and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]aminoN-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives.

Particular, preferred and specific values are the appropriate values mentioned above.

Compounds of the invention which are of particular interest include the compounds described in the accompanying Examples (and their pharmaceutically-acceptable salts), and are hence provided as a further feature of the present invention. In particular, the present invention provides a compound of formula I, or a pharmaceutically acceptable salt thereof, as described in Example 2, 3 or 5.

A suitable pharmaceutically-acceptable salt of the present invention comprises an acid-addition salt derived from an inorganic or organic acid which provides a pharmaceutically-acceptable anion. Thus, examples of salts of the present invention include acid-addition salts with hydrochloric, hydrobromic, nitric, sulphuric, phosphoric, trifluoroacetic, citric, tartaric, succinic, maleic, fumaric or acetic acid. In addition, suitable pharmaceutically-acceptable salts include [where the compound of formula I is sufficiently acidic, for example where the compound of formula I bears an acidic substituent such as carboxy] those formed with a base which affords a pharmaceutically acceptable cation. Suitable bases include an alkali metal salt (such as a sodium or potassium salt), an alkaline earth metal salt (such as a calcium or magnesium salt), an ammonium salt or a salt with an organic base which affords a physiologically-acceptable cation such as a salt with methylamine, dimethylamine, triethylamine, piperidine or morpholine.

The compounds of the present invention may be obtained by standard procedures of organic chemistry already known to be applicable to the preparation of structurally analogous compounds. Such procedures for the preparation of the compounds of formula I, or pharmaceutically acceptable salts thereof, are provided as a further feature of the present invention and are illustrated by the following preferred processes in which the various generic radicals, for example, R¹, R², Ar¹ and Ar² have any of the meanings defined hereinbefore, and in which Ar¹ and Ar² may be unsubstituted or substituted as hereinbefore defined.
(a) For those compounds in which R¹ is hydroxy and R² is hydrogen, reacting an organometallic derivative of formula III in which M is a metal atom or a derivative thereof, with quinuclidin-3-one.
   Suitable values for M include, for example, magnesium and lithium. In the case where M is magnesium it is conveniently present in the form of a derivative of formula -MgX where X is a halogen atom such as iodo or bromo, so that the organometallic compound of formula III is in the form known as a Grignard Reagent. The reaction is generally carried out in an inert solvent such as dry diethyl ether or tetrahydrofuran, with cooling. For example, the reaction may be carried out at a temperature from about ambient temperature to the reflux temperature of the reaction mixture.
   In cases where X is -COCH₂- or -CH₂CO- the carbonyl group may be protected as, for example, a ketal during the reaction of the compound of formula III with quinuclidin-3-one. The protecting group may then be removed using methods well known in the art.
   The compounds of formula III may be prepared from a compound of formula IIIa in which "hal" is a halogen atom, such as iodo or bromo. The compound of formula IIIa may be reacted directly with the metal M. Thus in the case of magnesium, the Grignard Reagent of formula III may be prepared by reaction of a compound of formula IIIa in which "hal" is bromo or iodo with magnesium turnings in an inert solvent such as diethyl ether, as is well known in the art. Where H is lithium, the compound of formula III may be prepared by reaction of the compound of formula IIIa with lithium in an inert solvent such as diethyl ether, or by reaction with an alkyl lithium derivative such as sec-butyl lithium in an inert solvent such as diethyl ether or tetrahydrofuran, as is well known in the art.
   The compounds of formula IIIa are generally available or may be readily prepared by procedures well known in the art. For example, compounds of formula Ar²-X-Ar¹-hal in which X is -OCH₂- may be prepared by reaction of a compound of formula Ar¹-OH with a compound of formula hal-Ar²-CH₂-hal ("hal" is halogen such as bromo) in the presence of a base. Suitable reaction conditions include the use of potassium carbonate in a solvent such as methylethylketone, preferably with heating to about 80°C. Compounds of formula IIIa in which X is -SCH₂- may be prepared in an analogous fashion. Thus compounds of formula IIIa in which X is -SCH₂- may be prepared by reaction of a compound of formula Ar¹-SH with a compound of formula hal-Ar²-CH₂-hal ("hal" is halogen such as bromo) in the presence of a base such as sodium hydride and a solvent such as dimethylformamide. Compounds of formula IIIa in which X is -CH₂O- and -CH₂S- may be prepared in an analogous manner, that is by reaction of a compound of formula Ar¹-CH₂-hal with a compound of formula hal-Ar²-OH or hal-Ar²-SH respectively.
   Compounds of formula IIIa in which X is -CH₂NH- may be prepared by reaction of a compound of formula hal-Ar¹-CHO (hal is halogen such as bromo) with a compound of formula Ar²-NH₂, followed by reduction using, for example, sodium borohydride in methanol. Similarly, compounds of formula IIIa in which X is -NHCH₂- may be prepared by reaction of a compound of formula hal-Ar¹-NH₂ with a compound of formula Ar²-CHO, followed by reduction.
   Compounds of formula IIIa in which X is -CH=CH- may be prepared by reaction of a compound of formula hal-Ar¹-CHO (hal is halogen such as bromo) with a compound of formula Ar²-CH₂-P⁺Ph₃Cl⁻ using conditions known to those skilled in the art for carrying out Vittig reactions, such as in the presence of potassium t-butoxide in a solvent such as tetrahydrofuran and with cooling. Similarly, they may be prepared by reaction of a compound of formula Ar¹-CH₂-P⁺Ph₃Cl⁻ with a compound of formula Ar²-CHO.
   Compounds of formula IIIa in which X is -CH₂CO- may be prepared by reaction of a compound of formula hal-Ar¹-CH₂COCl with a compound of formula Ar² using Freidel Crafts conditions (eg, AlCl₃). -The carbonyl group may then be protected by reaction with an alcohol such as (MeO)₃H. Compounds of formula IIIa in which X is -COCH₂- may be prepared by reaction of a compound of formula hal-Ar¹-hal (hal is halogen such as bromo) with a compound of formula Ar²-CH₂CN in the presence of butyllithium and a solvent such as tetrahydrofuran. The carbony group may then be protected by reaction with an alcohol.
   Compounds of formula IIIa in which X is -C≡C- may be prepared by reaction of a compound of formula hal-Ar¹-hal (hal is halogen such as bromo) with a compound of formula Ar²-C≡CH in the presence of CuI, or Pd(Ph₃P)₂Cl₂, Et₃N and a solvent such as dimethylformamide.
   Compounds of formula IIIa in which X is -CH₂CH₂- may be prepared by, for example, reduction of a compound of formula IIIa in which X is -C≡C- or -CH=CH- (eg. catalytic hydrogenation).
   Compounds of formula IIIa in when X is -O- or -S- may be prepared by reaction of a compound of formula hal-Ar¹-XH (hal is halogen such as bromo, X is -O- or -S-) with a compound of formula Ar²-Z (Z is a leaving group such as bromo, tosyl or triflate) in the presence of a base such as sodium hydride and a solvent such as dimethylformamide. Compounds of formula IIIa in which X is -NH- may be prepared by reaction of a compound of formula Ar²-Z (Z is a leaving group such as bromo, tosyl or triflate) in a solvent such as dimethylformamide and 2,2-dichlorobenzene with heating (eg. 50°C to reflux temperature of the reaction mixture). Alternatively, a compound of formula hal-Ar¹-NHCOCF₃ (hal is halogen such as bromo) may be reacted with a compound of formula Ar²-Z (Z is a leaving group such as bromo, tosyl or triflate) in the presence of a base such as sodium hydride and in a solvent such as dimethylformamide, follwed by treatment with sodium carbonate solution. Compounds of formula IIIa in which X is -CH₂- may be prepared by reaction of a compound of formula hal-Ar¹-hal (hal is halogen such as bromo) with a compound of formula Ar²-CHO in the presence of butyllithium and a solvent such as tetrahydrofuran with cooling (eg. at a temperature from -70°C to 0°C) to give a compound of formula IIIa in which X is -CH(OH)- which may be treated with Et₃SiH and trifluoroacetic acid to give the compound in which X is -CH₂-; or oxidised using, for example, manganese dioxide, to give the compound in which X is -CO-.
(b) For those compounds of formula I in which X is -CH=CH-, reacting a compound of formula IV in which Y is -CHO with a compound of formula V in which W is a halogen atom (such as chloro), in the presence of a base.
   Suitable bases include alkoxides such as potassium t-butoxide and the reaction is conveniently carried out in an inert solvent such as tetrahydrofuran with cooling below ambient temperature.
   The compounds of formula V may be prepared by rection of the appropriate benzylhalide with triphenylphosphine.
(c) For those compounds of formula I in which X is -C≡C-, reacting a compound of formula IV in which Y is a leaving group with a compound of formula VI in which M is a metal atom.
   Suitable leaving groups include, for example, halogen such as bromo, chloro or iodo.
   A suitable metal atom is lithium and the reaction is generally carried out in an inert solvent such as diethyl ether or tetrahydrofuran with cooling. The compounds of formula VI may be prepared by reaction of the metal with the appropriate phenylacetylene compound.
(d) For those compounds of formula I in which X is -OCH₂-, _NHCH₂- or -SCH₂- reacting a compound of formula IV in which Y is -OH, -NH₂ or -SH with a compound of formula VII in which Z is a leaving group, in the presence of a base.
   Suitable values for Z include halogen such as chloro, bromo or iodo; whilst suitable bases include alkali metal hydroxides such as sodium hydroxide, alkaline earth metal carbonate such as potassium carbonate and metal hydrides such as sodium hydride. The reaction is generally carried out in a solvent such as methylethylketone or dimethylformamide.
(e) For those compounds of formula I in which X is -CH₂O-, -CH₂NH- or -CH₂S- by reaction a compound of formula IV in which Y is -CH₂OH, -CH₂NH₂ or -CH₂SH with a compound of formula VIII in which Z is leaving group in the presence of a base.
   Suitable values for Z include, for example, halogen such as chloro or bromo, trifluoromethanesulphonyloxy, methanesulphonyloxy and tolunemethanesulphonyloxy. Suitable bases include, for example sodium hydride or alkoxides such as sodium butoxide. The reaction is generally carried out in a solvent such as dimethylformamide.
   The compounds of formula IV may be prepared by reaction of the corresponding compound of formula Y-Ar¹-M in which M represents a metal atom or derivative such as Lithium or -MgBr, with quinuclidin-3-one. The reaction is generally carried out in an inert solvent such as diethyl ether or tetrahydrofuran, and the compoud of formula Y-Ar¹-M may be prepared by reaction of the corresponding bromo compound of formula Y-Ar¹-Br with the metal. When the group Y is an -OH or -CH₂OH group it is generally protected during the reaction as, for example a silyl derivative; whilst when Y is -CHO it is generally protected as an acetal.
(f) For those compounds of formula I in which X is -S-, -SCH₂- or -CH₂S- wherein the sulphur atom bears one or two oxygen atoms, oxidising the corresponding compound of formula I in which X is -S-, -SCH₂- or -CH₂S-.
   The compounds of formula I in which X is -SCH₂- may be be oxidised to these in which the sulphur atom bears an oxygen atom (that is to a "sulphoxide") using, for example an appropriate quantity of sodium periodate. Further oxidation to the compound in which the sulphur atom bears two oxygen atoms (that is a "sulphone") may be carried out using a peracid such as peracetic acid or hydrogen peroxide. The oxidation of sulphur compounds to the corresponding sulphoxides and sulphones is well known in the chemical art. Compounds of formula I in which X is -CH₂S- may be oxidised to the corresponding sulphoxides or sulphones in the same way.
   In some cases oxidation of compounds of formula I to give a sulphone may be accompanied by some oxidation of the nitrogen atom in the quinuclidine ring to the N-oxide. In such cases the quinuclidine N-oxide moiety may be reduced back to a quinuclidine moiety without affecting the sulphone using reducing agents well known in the art, such as sulphur dioxide.
   It will be appreciated that the compounds of formula I may also be prepared by forming the link (X) between Ar¹ and Ar² after coupling Ar¹ to the quinuclidine moiety. Such methods are analogous to these described above.
   It will be appreciated that in some of the reactions mentioned herein it may be necessary/desirable to protect any sensitive groups in the compounds. The instances where protection is necessary or desirable and suitable methods for protection are known to those skilled in the art. Thus, if reactants include groups such as amino, carboxy or hydroxy it may be desirable to protect the group in some of the reactions mentioned herein. Suitable protecting groups for hydroxy include, for example, silyl groups such as trimethylsilyl or t-butyldimethylsilyl, tetrahydropyranyl and esterifying groups such as a methyl or ethyl ester; and for amino groups include benzyloxycarbonyl and t-butoxycarbonyl. Carboxy groups may be protected in a reduced form such as in the form of the corresponding protected alcohol, which may be subsequently oxidised to give the carboxy group. The protecting groups may be removed at any convenient stage in the synthesis using conventional techniques well known in the chemical art.
   It will also be appreciated that the preferred process for preparing a particular compound of formula I will depend upon the nature of the various radicals. Similarly, the preferred choice of reagent will depend upon the nature of the various radicals present. For example, when it is required to reduce a particular compound the reducing agent will generally be selected to be one which does not interfere with other groupings present.
   It will also be appreciated that certain of the various optional substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acylhalide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.
   When a pharmaceutically-acceptable salt of a compound of the formula I is required, it may be obtained, for example, by reaction of said compound with the appropriate acid (which affords a physiologically acceptable anion), or with the appropriate base (which affords a physiologically acceptable cation), or by any other conventional salt formation procedure.
   As mentioned previously, the compounds of the formula I (and their pharmaceutically-acceptable salts) are inhibitors of the enzyme squalene synthase. Thus the compounds of the present invention are capable of inhibiting cholesterol biosynthesis by inhibition of de novo squalene production.
   The beneficial pharmacological properties of the compounds of the present invention may be demonstrated using one or more of the following techniques.

### (a) Inhibition of Squalene synthase

In this test, the ability of a compound to prevent the formation of squalene from a radioactive substrate (tritiated farnesyl pyrophosphate) is assessed.

The test compound is incubated at a concentration of 25 micromolar in 200µl of a buffered solution containing potassium phosphate (50mM), MgCl₂ (4.95mM), KF (9.9mM), NADPH (0.9mM) and rat liver microsomal protein (20µg). Rat liver microsomes are prepared by the method described in published European Patent Application No. 324,421 and stored in liquid nitrogen prior to assay. Assay vials are kept at 37°C throughout the incubation.

The reaction is started with the addition of the substrate (1-[³H]-farnesyl pyrophosphate), final concentration 20µM, and stopped after 15 minutes reaction time with the addition of 50µl of 4% KOH. The reaction products are separated from unreacted substrate after application to a C-18 octadecyl lccBond column (Analytichem Int product No. 617101). An aqueous fraction is eluted with 250µl of 0.1M KOH. Squalene is then eluted with 1.0 ml 10% ethylacetate in hexane and radioactivity determined. The difference in radioactivity in the presence and absence of the test compound is used to determine the level of inhibition. If the test compound inhibits at greater than about 70% at 25 micromolar, it is generally re-tested at 25 and 2.5 micromolar. The IC₅₀ (concentration which results in a 50% inhibition of squalene production), of the test compound can be determined by testing the compound at several, for example five, concentrations predicted from the two concentration results. The IC₅₀ can then be determined from a plot of percentage inhibition against concentration of test compound.

In general, compounds of formula I show significant inhibition in the above test at a concentration in the range of about 0.001 to 25µM.

By way of illustration of the squalene synthase inhibitory properties of the compound of formula I, described in Example 2 below gave an inhibition of about 89% at 2.5µM.

### (b) Acute rat cholesterol synthesis assay.

This is an acute in vivo test in the rat to measure de novo hepatic cholesterol synthesis from exogenously administered ¹⁴C-acetate.

Female rats (35 - 55 g) are housed in reverse lighting conditions (red light from 0200h - 1400h) for a period of about 2 weeks prior to test. Animals are allowed free access to chow and drinking water throughout this period. At test, animals should weigh 125 - 150 g.

Test compounds may be administered by oral gavage, dissolved or suspended in 0.5% polysorbate, or by ip or iv dosing. Control animals receive vehicle alone. After 1 hour the rats are injected ip with 25µCi [2-¹⁴C]-acetate (NEN DUPONT. specific activity, 45-60mCi/mmol NEC-085H, or AMERSHAM specific activity, 50-60mCi/mmol CFA 14) in a volume of 0.25 ml saline (100µCi/ml). After a further hour, rats are terminally anaesthetised with halothane and a blood sample obtained from the abdominal vena cava.

1ml of plasma is lyophilised and then saponified in 2ml ethanolic KOH (1 part 33% KOH, 9 parts ethanol) at 75°C for 2 hours. After addition of an equal quantity of water, non-saponifiable lipids are extracted with two 5ml volumes of hexane. The hexane extracts are evaporated to dryness and the residues dissolved in ethanol to determine cholesterol specific radioactivity. ED₅₀ values can be determined in the standard way.

In general, compounds of formula I show activity in the range of about 0.1 to 100 mg/kg.

By way of illustration, the compound of formula I described in Example 2 gave a 72% reduction in the rate of cholesterol biosynthesis at a concentration of 50mg/kg.

In an alternative in vivo test, de novo hepatic cholesterol synthesis from exogenously administered 3H-mevalonolactone is measured. The above procedure is used but with ³H-mevalonolactone (2.5 µCi) adminsitered in place of ¹⁴C-acetate and the test compound is generally adminstered as a solution or suspension in 10% dimethylsulphoxide in 0.5% hydroxypropylmethylcellulose.

No overt toxicity was detected when compounds of the formula I were administered at several multiples of their minimum inhibitory dose or concentration.

As mentioned above, the compounds of the present invention are squalene synthase inhibitors and hence possess the property of inhibiting cholesterol biosynthesis. Thus the compounds of the present invention will be useful in treating diseases or medical conditions in which an inhibition of squalene synthase is desirable, for example those in which a lowering of the level of cholesterol is blood plasma is desirable. In particular, the compounds of the present invention will be useful in treating hypercholesterolemia and/or ischaemic diseases associated with atheromatous vascular degeneration such as atherosclerosis. The compounds of the present invention will also be useful in treating fungal infections.

Thus according to a further feature of the present invention there is provided a method of inhibiting squalene synthase in a warm-blooded animals (such as man) requiring such treatment, which method comprises administering to said animal an effective amount of a compound of formula I (as herein defined), or a pharmaceutically-acceptable salt thereof. In particular, the present invention provides a method of inhibiting cholesterol biosynthesis, and more particularly to a method of treating hypercholesterolemia and atheromatous vascular degeneration (such as atherosclerosis).

Thus the present invention also provides the use of a compound of formula I (as herein defined), or a pharmaceutically-acceptable salt thereof, for the manufacture of a medicament for treating diseases or medical conditions in which a lowering of the level of cholesterol in blood plasma is desirable (such as hypercholesterolemia and atherosclerosis).

When used in the treatment of diseases and medical conditions in which an inhibition of cholesterol biosynthesis is desired, for example in the treatment of hypercholesterolemia or atherosclerosis, it is envisaged that a compound of formula I (or a pharmaceutically acceptable salt thereof) will be administered orally, intravenously, or by some other medically acceptable route so that a dose in the general range of, for example, 0.01 to 50 mg per kg body weight is received. However it will be understood that the precise dose administered will necessarily vary according to the nature and severity of the disease, the age and sex of the patient being treated and the route of administration.

In general, the compounds of formula I (or a pharmaceutically-acceptable salt thereof) will usually be administered in the form of a pharmaceutical composition, that is together with a pharmaceutically acceptable diluent or carrier, and such a composition is provided as a further feature of the present invention.

A pharmaceutical composition of the present invention may be in a variety of dosage forms. For example, it may be in the form of tablets, capsules, solutions or suspensions for oral administration, in the form of a suppository for rectal administration; in the form of a sterile solution or suspension for parenteral administration such as by intravenous or intramuscular injection.

A composition may be obtained by conventional procedures using pharmaceutically acceptable diluents and carriers well known in the art. Tablets and capsules for oral administration may conveniently be formed with a coating, such as an enteric coating (for example, one based on cellulose acetate phthalate), to minimise dissolution of the active ingredient of formula I (or a pharmaceutically-acceptable salt thereof) in the stomach or to mask unpleasant taste.

The compounds of the present invention may, if desired, be administered together with (or sequentially to) one or more other pharmacological agents known to be useful in the treatment of cardiovascular disease, for example, together with agents such as HHG-CoA reductase inhibitors, bile acid sequestrants, other hypocholesterolaemic agents such as fibrates, for example gemfibrozil, and drugs for the treatment of coronary heart disease. As a further example, the compounds of the present invention may, if desired, be administered together with (or sequentially to) an angiotensin converting enzyme (ACE) inhibitor, such as captopril, lisinopril, zofenopril or enalapril.

Compounds which inhibit squalene synthase have also found utility as antifungal agents. Thus the present invnetion also provides a method of treating fungal infections comprising adimistering a compound of formula I, or a non-toxic salt thereof, to an organism in need of such treatment.

In particular, the present invention also provides a method of treating fungal infections which comprises administration to an a warm blooded animal, such as man, in need of such treatment an effective amount of a compound of formula I, or a pharmaceutically acceptable salt thereof.

When used as anti-fungal agents the compounds may be formulated in a variety of ways, the nature of such formulation depending on whether the use is for controlling pathogens infecting mammals such as man, or in agriculture such as in soil or plants, or some other object. For medical applications, the compounds of the present invention may, in -addition to the formulations mentioned above, be adapted for topical administration and such a composition is provided as a further feature of the present invention. Such compositions may be in a variety of forms, for example creams or lotions.

The invention will now be illustrated by the following non-limiting Examples in which, unless otherwise stated:-
(i) evaporations were carried out by rotary evaporation in vacuo;
(ii) operations were carried out at room temperature, that is in the range 18-26°C;
(iii) flash column chromatography or medium pressure liquid chromatography (MPLC) was performed on silica gel (Merck Kieselgel Art.9385, obtained from E Merck, Darmstadt, Germany);
(iv) yields are given for illustration only and are not necessarily the maximum attainable by diligent process development;
(v) proton NMR spectra were normally determined at 200 MHz using tetramethylsilane (TMS) as a internal standard, and are expressed as chemical shifts (delta values) in parts per million relative to TMS using conventional abbreviations for designation of major peaks: s, singlet; m, multiplet; t, triplet; br, broad; d, doublet;
(vi) all end-products were characterised by microanalysis, NMR and/or mass spectroscopy (m/z values); and
(vii) conventional abbreviations are used for individual radicals and recrystallisation solvents, for example, He = methyl, Et = ethyl, Pr = Propyl, Prⁱ = isopropyl, Bu = butyl, Buⁱ = isobutyl, Ph = phenyl; EtOAc = ethyl acetate, Et₂O = ether, MeCN = acetonitrile, MeOH = methanol, EtOH = ethanol, PrⁱOH = 2-propanol, H₂O = water.

### EXAMPLE 1

A solution of sec-butyllithium in cyclohexane (1.74 ml, 1.3M) was added dropwise with stirring to a solution of 2-(4-bromophenoxy)-pyridine (477 mg) in dry tetrahydrofuran (10 ml) under an atmosphere of argon at -78°C. The mixture was stirred for 5 minutes and a solution of quinuclidin-3-one (214 mg) in dry tetrahydrofuran (5 ml) added over a period of 1 minute. Stirring was continued at -78°C for 30 minutes and the mixture was then allowed to reach room temperature over a period of 2 hours. Water (15 ml) was then added to the reaction mixture whilst keeping the reaction temperature below 10°C. The aqueous layer was extracted with ethyl acetate, the ethyl acetate phase separated, dried (Na₂SO₄) and evaporated to give a residue which was purified by medium pressure column chromatography on alumina (ICN Alumina N 32-63), eluting with 10% methanol in ethyl acetate. The residue obtained was further purified by recrystallisation from ethyl acetate to give 3-[4-(2-pyridyloxy)phenyl]quinuclidin-3-ol as a colourless solid, m.p. 167-170°C; microanalysis, found: C, 73.1; H, 7.0; N, 9.0%; C₁₈H₂₀N₂O₂ requires: C, 72.0; H, 6.8; N, 9.4%; NMR (DMSOd₆): 1.3-1.5(3H, m), 1.9(1H, s), 2.1-2.2(1H, m), 2.6-2.8(4H, m), 2.9(2H, d) 3.4(2H, d), 5.1(1H, s), 7.0-7.2(4H, m), 7.5-7.6(2H, d), 7.8-7.9(1H, m) and 8.1-8.2(1H, d of d); m/Z 297 (M+H).

The preparation of 2-(4-bromophenoxy)pyridine is described, for example, in J.O.C., (1949), 14, 783.

Thus the 2-(4-bromophenyloxy)pyridine used as starting material was prepared as follows.

4-Bromophenol (1.73g) was added to a stirred suspension of sodium hydride (440mg of a 60% suspension in mineral oil) in dry dimethylformamide (10ml). After 10 minutes, a solution of 2-bromopyridine (1.66g) in dry dimethylformamide (10ml) was added over a period of 10 minutes. The reaction mixture was heated at 100°C for 3 days. The reaction mixture was cooled to room temperature, poured onto water (400ml) and the aqueous layer extracted with diethyl ether. The diethyl ether phase was separated, dried (Na₂SO₄) and evaporated to give a residue which was purified by medium pressure column chromatography on silica gel using 5% ethyl acetate in hexane as eluent to give 2-(4-bromophenyloxy)pyridine (510mg) as a colourless oil; NMR(CDCl₃): 6.9-7.0(2H,m), 7.0-7.1(2H,d), 7.5-7.6(2H,d), 7.65-7.75(1H, d of t) and 8.2(1H,d).

### EXAMPLE 2

A solution of sec-butyllithium in cyclohexane (5.12 ml, 1.3M) was added dropwise to a stirred solution of (E)-3-[2-(4-bromophenyl)-vinyl]pyridine (1.57 g) in dry tetrahydrofuran (27 ml) under an atmosphere of argon at-78°C. The mixture was stirred for 5 minutes and a solution of quinuclidin-3-one (720 mg) in dry tetrahydrofuran (14 ml) added over a period of 3 minutes. Stirring was continued at -78°C for 30 minutes and the mixture was then allowed to reach room temperature over 2 hours. Water (50 ml) was then added to the reaction mixture whilst keeping the reaction temperature below 10°C. The aqueous layer was extracted with ethyl acetate, the ethyl acetate phase separated, dried (Na₂SO₄) and evaporated to give a residue which was purified by medium pressure column chromatography on alumina (ICN Alumina N 32-63), eluting with 5% methanol in ethyl acetate. The residue obtained was further purified by recrystallisation from ethyl acetate/hexane to give, as a colourless solid, 3-(4-[(E)-2-(3-pyridyl)vinyl]phenyl)quinuclidin-3-ol (400 mg), m.p. 160-161°C, microanalysis, found: C, 77.9; H, 7.5; N, 8.7%; C₂₀H₂₂N₂0.0.1H₂O requires: C, 77.7; H, 7.2; N, 9.1%; NMR. (CDCl₃):
1.4-1.6(3H, m), 2.2-2.3(2H, m), 2.7-2.8(2H, t), 2.85-3.05(2H, m), 3.05-3.15(1H, d), 3.45-3.55(1H, d of d), 7.1(2H, d), 7.3(1H, m), 7.5(4H, s), 7.8(1H, d of t), 8.45(1H, d of d) and 8.6(1H, d of d); m/Z 307 (M+H).

The (E)-3-[2-(4-bromophenyl)vinyl]pyridine used as starting material was prepared as follows:- Potassium-tert-butoxide (670 mg) was added to a stirred suspension of 4-bromobenzyltriphenylphosphonium bromide (2.56 g) in dry tetrahydrofuran (25 ml) under an atmosphere of argon. After 20 minutes, the reaction mixture was cooled to 5°C and a solution of 3-pyridine carboxaldehyde (560 mg) in dry tetrahydrofuran (10 ml) was added over a period of 20 minutes. Stirring was continued at 5°C for 30 minutes and the mixture was then allowed to reach room temperature over a period of 2 hours. Water (75 ml) was then added to the reaction mixture whilst keeping the reaction temperature below 10°C. The aqueous layer was extracted with diethyl ether, the diethyl ether phase separated, dried (Na₂SO₄) and evaporated to give a residue which was purified by medium pressure column chromatography on silica gel, eluting with 30% ethyl acetate in hexane to give (Z)-3-[2-(4-bromophenyl)vinyl]pyridine (770 mg) as a colourless oil; NMR (CDCl₃): 6.5-6.7(2H, d of d), 7.0-7.1(2H, d), 7.1-7.2(1H, m), 7.3-7.4(2H, d), 7.5(1H, d) and 8.4-8.5(2H, m). Further elution with the same solvent mixture gave, as a colourless solid, (E)-3-[2-(4-bromophenyl)vinyl]pyridine (400 mg), m.p. 96-97°C; NMR (CDCl₃): 7.1(2H, s), 7.3(1H, m), 7.35-7.5(4H, d of d), 7.8(1H, d) and 8.5-8.8(2H, m).

### EXAMPLE 3

Using a similar procedure to that in Example 2 but using (Z)-3-[2-(4-bromophenyl)vinyl]pyridine as starting material (in place of (E)-3-[2-(4-bromophenyl)vinyl]pyridine) there was obtained 3-(4-[(Z)-2-(3-pyridyl)vinyl]phenyl)quinuclidin-3-ol (12% yield), m.p. 167-169°C, microanalysis, found: C, 78.2; H, 7.3; N, 8.8%; C₂₀H₂₂N₂O requires: C, 78.4; H, 7.2; N, 9.1%; NMR: (CDCl₃): 1.3-1.5(3H, m), 2.0-2.1(1H, m), 2.1-2.3(1H, m), 2.65-3.0(5H, m), 3.1(1H, d of d), 6.5-6.8(2H, d of d), 7.1(1H, d), 7.15-7.4(4H, d of d), 7.5(1H, d of t), 8.3(1H, d of d) and 8.35(1H, d): m/z 307 (M+H).

### EXAMPLE 4

A solution of tert-butyllithium in pentane (2.4ml, 1.7M) was added dropwise with stirring over 10 minutes to a solution of 2-(4-bromophenyloxy)quinoline (600mg) in dry tetrahydrofuran (20ml) under an atmosphere of argon at -78°C. The mixture was stirred for 30 minutes and a solution of quinuclidin-3-one (225mg) in dry tetrahydrofuran (5ml) was then added over a period of 15 minutes. Stirring was continued for a further 3 hours at -78°C and the mixture was then allowed to reach room temperature over a period of 30 minutes. The mixture was added to water and extracted with ethyl acetate. The ethyl acetate phase was separated, dried (MgSO₄) and evaporated to give an oil. The oil was dissolved in ethyl acetate (4ml) and hexane (10ml) was added to the solution to precipiate a solid. This solid was crystallised from ethyl acetate to give 3-[4-(2-quinolyloxy)phenyl)quinuclidin-3-ol as a colourless solid (244mg), mp. 266-266.5°C; microanalysis, found: C, 75.6; H, 6.50; N, 7.80%; C₂₂H₂₂N₂O₂ 0.1H₂O requires: C, 75.8; H, 6.38; N, 8.04%; NMR ([DHSO-d₆): 1.25-1.60(3H,m); 1.99(1H,m); 2.05-2.28(1H,m); 2.60-2.85(4H,m); 2.95(1H,d); 3.40(1H,d); 5.21(1H,s); 7.15-7.27(3H,m); 7.42-7.52(1H,m); 7.52-7.67(4H,m); 7.94(1H,d) and 8.41(1H,d); m/z 347(M+H).

The 2-(4-bromophenyloxy)quinoline used as starting material was prepared in the following manner.

Sodium hydride (0.86g; 60% dispersion in oil) was added to a stirred solution of 4-bromophenol (3.46g) in dimethylformamide (30ml) at room temperature under an atmosphere of argon. The mixture was stirred for 10 minutes and a solution of 2-chloroquinoline (3.26g) in dimethylformamide (5ml) was then added over a period of 5 minutes. The mixture was heated at 135°C for 4 hours. The mixture was allowed to cool to ambient temperature and was then added to ice-water to give a pale grey solid. The solid, was dried and then crysallised from hexane to give 2-(4-bromophenyloxy)quinoline (1.83g) as a colourless solid, m.p.112-3°; microanalysis, found: C, 59.7; H, 3.30; N, 4.60%; C₁₅H₁₀BrNO requires: C, 60.0; H, 3.36; N, 4.67%.

### EXAMPLE 5

Using a similar procedure to that in Example 2 but using (E)-[4-(3,4-methylenedioxyphenyl)vinyl]bromobenzene as starting material, there was obtained 3-(4-[(E)-2-(3,4-methylenedioxyphenyl)vinyl]phenyl)-quinuclidin-3-ol (52% yield) as a solid, m.p. 206-207°C, microanalysis, found: C, 75.3; H, 6.6; N, 3.9%; NMR(DMSOd₆/CD₃COOD): 1.5-1.7(1H,m), 1.8-1.9(2H,m), 2.4-2.5(2H,m), 3.2-3.4(4H,m), 3.4-4.0(2H, d of d), 6.0(2H,s); 6.8-6.9(1H,d); 7.0-7.1(1H, d of d); 7.1-7.2(2H,d); 7.2(1H, d of d) and 7.5-7.6(4H,m): m/z 350 (M+H).

The (E)-[4-(3,4 methylenedioxyphenyl)vinyl]bromobenzene used as starting material was prepared in a similar procuedure to that described in Example 2 for the preparation of (E)-3-[2-(4-bromophenyl)vinyl]-pyridine but using piperonal in place of 3-pyridinecarboxyaldehyde. There was obtained (E)-[4-(3,4-methylenedioxyphenyl)vinyl]bromobenzene (27% yield) as a solid, m.p. 139-140°C, microanalysis, found: C, 59.2; H, 3.5%; C₁₅H₁₁BrO₂ requires: C,59.4; H, 3.7%; NMR(CDCl₃): 6.0(2H,s) and 6.8-7.5(9H,m); m/z 304 (M+H).

### EXAMPLE 6

Using a similar procedure to that in Example 2 but using (E)-2-[2-(4-bromophenyl)vinyl]-1-methylpyrrole as starting material in place of 4-(2-pyridyloxy)bromobenzene) there was obtained 3-(4-[(E)-2-(1-methylpyrrol-2-yl])vinyl]phenyl)quinuclidin-3-ol (36% yield) as a solid, m.p. 158-160°C, microanalysis, found: C, 77.8; H, 8.0; N, 8.8%; C₂₀H₂₄N₂O requies; C, 77.9; H, 7.8; N, 9.1%; NMR(DMSO-d₆): 1.2-1.5(3H,m), 1.9-2.0(1H,m), 2.1-2.2(1H,m), 2.6-2.8(5H,m), 2.9-3.4(2H, d of d), 3.7(3H,s), 5.0(1H,s), 6.0(1H, d of d), 6.5(1H, d of d), 6.8(1H,t), 6.8-7.2(2H, d of d) and 7.5(4H,m); m/z 309 (M+H).

The (E)-2-[2-(4-bromophenyl)vinyl]1-methylpyrrole used as starting material was prepared in a similar procedure to that described in Example 2 for the preparation of (E)-3-[2-(4-bromophenyl)vinyl]pyridine but using 1-methylpyrrole-2-carboxaldehyde (in place of 3-pyridinecarboxaldehyde). There was thus obtained (E)-2-[2-(4-bromophenyl)vinyl]-1-methylpyrrole (20% yield) as a solid, m.p. 93-94°C; microanalysis, found: C, 59.3; H, 4.5; N, 5.0%, C₁₃H₁₂BrN requires: C,59.6; H, 4.6; N,5.3%; NMR(CDCl₃): 3.7(3H,s), 6.1(1H,t), 6.5(1H,d), 6.6(1H,d), 6.7-7.0(1H, d of d) and 7.3-7.5(4H,d of d) m/z 263 (M+H).

### EXAMPLE 7

Using a similar procedure to that in Example 2 but using (E)-2-[2-(4-bromophenyl)vinyl]thiophene as starting material (in place of 4-(2-pyridyloxy)bromobenzene) there was obtained 3-[4-((E)-2-(2-thienyl)vinyl)phenyl]quinuclidin-3-ol (16% yield) as a solid, m.p. 183-184°C, NMR:(DMSOd₆/CD₃COOD): 1.7-1.9(3H,m); 2.2-2.5(2H,m); 3.2-3.4(4H,m); 3.4-3.9(2H, d of d), 6.9-7.0(1H, d of d); 7.1-7.2(2H, d of d) and 7.3-7.6(6H,m); m/z 312 (M+H).

The (E)-2-[2-(4-bromophenyl)vinylthiophene used as starting material was prepared in a similar procedure to that described in Example 2, for the preparation of (E)-3-[2-(4-bromophenyl)vinyl]pyridine but using 2-thiophenecarboxaldehyde (in place of 3-pyridine carboxaldehyde). There was thus obtained (E)-2-[2-(4-bromophenyl)vinyl]thiophene (33% yield), m.p. 149-150°C, microanalysis, found: C, 54.5; H, 3.4%; C₁₂H₉BrS requires: C, 54.4; H, 3.4%; NMR(CDCl₃): 6.8-6.9(1H,d), 7.0(iH, d of d), 7.1(1H, d of d), 7.2-7.3(2H,m) and 7.3-7.5(2H, d of d); m/z 266(M+H).

### EXAMPLE 8

Using a similar procedure to that in Example 5 but using 2-(4-bromobenzyloxy)quinoline as starting material (in place of 4-(2-pyridyloxy)bromobenzene) there was obtained 3-[4-(2-quinolyloxymethyl)phenyl]quinuclidin-3-ol (37% yield) as a solid m.p. 160-161°C; microanalysis, found: C, 76.5; H, 7.0; N, 7.6%; C₂₃H₂₄N₂O₄ requires: C, 76.6; H, 6.7; N, 7.8%; NMR(DMSOd₆/CD₃COOD): 1.5-1.9(3H,m), 2.3-2.5(2H,m), 3.2-3.4(4H,m), 3.4-3.9(2H, d of d), 5.6(2H,s), 7.0-7.1(1H,d), 7.4-7.5(1H, d of t), 7.6(4H,s), 7.7(1H, d of t); 7.8-7.9(2H,m) and 8.2(1H,d); m/z 361 (M+H).

The 2-(4-bromobenzyloxy)quinoline used as starting material was prepared as follows.

2-Hydroxyquinoline (1.25g) was added to a stirred suspension of silver carbonate (1.32g) in dry toluene (50ml). After 10 minutes, 4-bromobenzylbromide (1.72g) was added and the reaction mixture stirred in the absence of light at room temperature for 16 hours. The reaction mixture was filtered. The filtrate was evaporated and the residue purified by medium pressure column chromatography on silica gel, using 5% ethyl acetate in hexane as eluent. The residue obtained was further purified by recrystallisation from hexane to give 2-(4-bromobenzyloxy)quinoline (79% yield) as a colourless solid, m.p. 95-97°C, microanalysis, found: C, 61.2; H,3.8; N, 4.5%; C₁₆H₁₂BrNO requires: C, 61.2; H, 3.8; N, 4.5%; NMR(CDCl₃): 5.5(2H,s), 6.9-7.0(1H,d), 7.3-7.5(5H,m), 7.5-7.7(2H,m), 7.8-7.9(1H,d) and 8.0(1H,d); m/z 315 (M+H).

### EXAMPLE 9

Illustrative pharmaceutical dosage forms suitable for presenting the compounds of the invention for therapeutic or prophylactic use include the following tablet and capsule formulations, which may be obtained by conventional procedures well known in the art of pharmacy and are suitable for therapeutic or prophylactic use in humans:-

| (a) **Tablet I** | |
|---|---|
| | **mg/tablet** |
| Compound Z* | 1.0 |
| Lactose Ph. Eur. | 93.25 |
| Croscarmellose sodium | 4.0 |
| Maize starch paste (5% w/v aqueous paste) | 0.75 |
| Magnesium stearate | 1.0 |

| | |
|---|---|
| Note * The active ingredient Compound Z is a compound of formula I, or a salt thereof, for example a compound of formula I described in any of the preceding Examples. | |

| (b) **Tablet II** | **mg/tablet** |
|---|---|
| Compound Z* | 50 |
| Lactose Ph. Eur | 223.75 |
| Croscarmellose sodium | 6.0 |
| Maize starch | 15.0 |
| Polyvinylpyrrolidone (5% w/v aqueous paste) | 2.25 |
| Magnesium stearate | 3.0 |

| | |
|---|---|
| Note * The active ingredient Compound Z is a compound of formula I, or a salt thereof, for example a compound of formula I described in any of the preceding Examples. | |

| (c) **Tablet III** | **mg/tablet** |
|---|---|
| Compound Z* | 100 |
| Lactose Ph. Eur. | 182.75 |
| Croscarmellose sodium | 12.0 |
| Maize starch paste (5% w/v aqueous paste) | 2.25 |
| Magnesium stearate | 3.0 |

| | |
|---|---|
| Note * The active ingredient Compound Z is a compound of formula I, or a salt thereof, for example a compound of formula I described in any of the preceding Examples. | |

| (d) **Capsule** | |
|---|---|
| | **mg/capsule** |
| Compound Z* | 10 |
| Lactose Ph.Eur. | 488.5 |
| Magnesium stearate | 1.5 |

| | |
|---|---|
| Note * The active ingredient Compound Z is a compound of formula I, or a salt thereof, for example a compound of formula I described in any of the preceding Examples. | |

The tablet compostions (a) - (c) may be enteric coated by conventional means, for example, with cellulose acetate phthalate.

### CHEMICAL FORMULAE

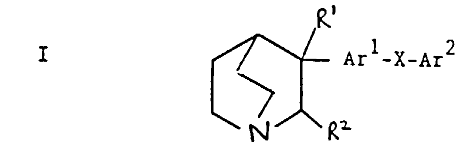

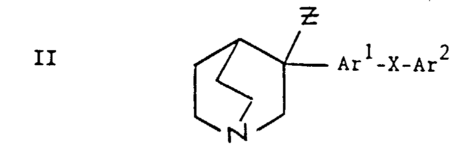

III Ar²-X-Ar¹-M

IIIa Ar²-X-Ar¹-hal

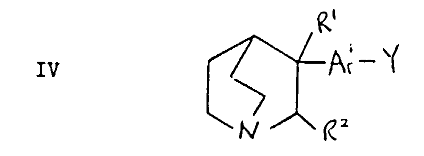

### CHEMICAL FORMULAE

V Ar²-CH₂P⁺Ph₃W⁻

VI Ar²-C≡CM

VII Z-CH₂Ar²

VIII Z-Ar²

## Claims

1. A compound of formula I, 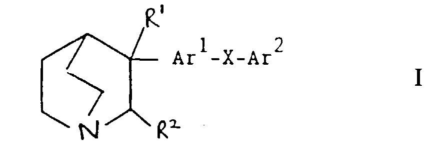 or a pharmaceutically acceptable salt thereof, wherein:
R¹ is hydroxy;
R² is hydrogen;
X is selected from -CH₂CH₂-, -CH=CH-, -C≡C-, -CH₂O-, -OCH₂-, -CH₂NH-, -NHCH₂₋, -CH₂CO-, -COCH₂₋, -CH=N-, -N=CH-, -O-, -NH-, -CO-, -CH₂₋, -CH₂S-, -SCH₂₋ and -S- (wherein the sulphur atom in the latter three groups may optionally bear one or two oxygen atoms);
Ar¹ is a phenylene moiety;
Ar² is a heterocyclic moiety;
and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino N-(1-6C)alkylcarbamoyl, di-N,N-[(1-6C)alkyl]carbamoyl, (1-6C)alkoxycarbonyl, (1-6C)alkylthio,(1-6C)alkylsulphinyl, (1-6C)alkylsulphonyl, halogeno (1-6C)alkyl, (1 -6C)alkanoylamino,ureido, N'-(1-6C)alkylureido, (1-6C)alkanoyl and oxime derivatives thereof and O-(1-6C)alkyl ethers of said oxime derivatives.

2. A compound as claimed in claim 1 wherein Ar¹ is a 1,4-phenylene moiety.

3. A compound as claimed in any one of the preceeding claims wherein one or both of Ar¹ and Ar² may optionally be unsubstituted or may bear one or more substituents independently selected from halogeno, hydroxy, amino, nitro, (1-6C)alkyl, (2-6C)alkenyl, (1-6C)alkoxy, (1-6C)alkanoyl and oxime derivatives thereof and O-(1-6C)alkyl ethers of said oximes, halogeno(1-6C)alkyl and (1-6C)alkanoylamino.

4. A compound as claimed in any one of the preceeding claims wherein Ar² is selected from furyl, pyrrolyl, thienyl, pyridyl, pyrimidinyl, pyridazinyl, imidazolyl, oxazolyl, isooxazolyl, thiazolyl, isothiazolyl, oxadiazolyl, 1,2,3-triazolyl, 1,2,4-triazolyl, benzfuranyl, quinolyl, isoquinolyl, benzimidazolyl, indolyl, benzthiazolyl, benzodioxolyl, benzodioxanyl and benzodihydrofuranyl.

5. A compound as claimed in in any one of the preceeding claims wherein X is selected from -CH₂CH₂₋, -CH=CH-, -C-C-, -CH₂O-, -OCH₂₋, -CH₂NH-, -NHCH₂₋, -CH₂CO-, -COCH_{2-,} -O-, -NH-, -CH₂S-, -SCH₂₋, -S- and wherein the sulphur atom in the latter three groups may optionally bear one or two oxygen atoms.

6. A compound as claimed in any one of the preceeding claims wherein Ar² is selected from 2-thienyl, 2-pyridyl, 3-pyridyl, 2-pyrrolyl, 2-imidazolyl, 1,2,3-triazol-4-yl, 1,2,4-triazol-3-yl, 3-quinolyl, 2-quinolyl, 2-thiazolyl, 5-thiazolyl, 1-isoquinolyl, 3-isoquinolyl, 2-benzimidazolyl, 2-benzthiazolyl, 5-oxadiazoyl, 2-indolyl and 3-indolyl, which may optionally bear one or two substituents independently selected from fluoro, chloro, bromo, iodo, hydroxy, nitro, methyl, ethyl, propyl, isopropyl, butyl, isobutyl, sec-butyl, allyl, methoxy, ethoxy, propoxy, isopropoxy, butoxy, formyl, acetyl propionyl, butyryl and oxime derivatives of the last four groups and O-methyl, ethyl, propyl, isopropyl and butyl ethers of said oximes, acetamido, propionamido, isopropionamido, fluoromethyl difluoromethyl and trifluoromethyl.

7. A compound as claimed in any one of the preceeding claims wherein X is selected from from -CH=CH-,-CH₂O- and -C≡C-.

8. A compound as claimed in claim 1 which is selected from:
3-(4-[(E)-2-(3-pyridyl)vinyl]phenyl)quinuclidin-3-ol;
3-(4-[(Z)-2-(3-pyridyl)vinyl]phenyl)quinuclidin-3-ol;
and their pharmaceutically acceptable salts.

9. A process for preparing a compound of formula I, or a pharmaceutially acceptable salt thereof, as claimed in claim 1 which process is selected from:
(a) for those compounds in which R¹ is hydroxy and R² is hydrogen, reacting an organometallic derivative of formula III:
Ar²-X-Ar¹-M (III)
in which M is a metal atom or a derivative thereof, with quinuclidin-3-one;
(b) for those compounds of formula I in which X is -CH=CH-, reacting a compound of formula IV: 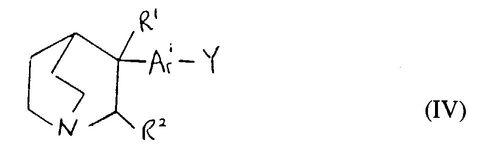 in which Y is -CHO with a compound of formula V in which W is a halogen atom (such as chloro), in the presence of a base;
(c) for those compounds of formula I in which X is -C≡C-, reacting a compound of formula IV: 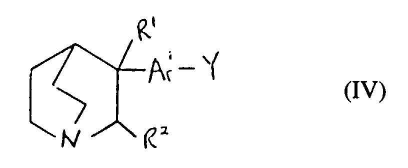 in which Y is a leaving group with a compound of formula VI:
Ar²-C≡CM (VI)
in which M is a metal atom;
(d) for those compounds of formula I in which X is -OCH₂₋, -NHCH₂₋ or -SCH₂₋ reacting a compound of formula IV: 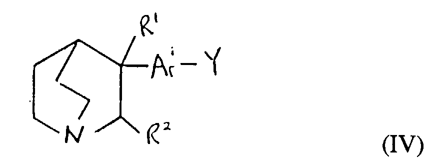 in which Y is -OH, -NH₂ or -SH with a compound of formula VII:
Z-CH₂Ar² (VII)
in which Z is a leaving group, in the presence of a base;
(e) for those compounds of formula I in which X is -CH₂O-, -CH₂NH- or -CH₂S- by reaction a compound of formula IV: 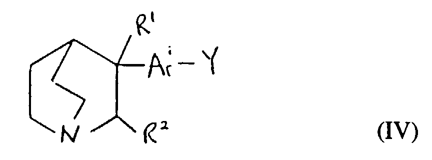 in which Y is -CH₂OH, -CH₂NH₂ or -CH₂SH with a compound of formula VIII:
Z-Ar² (VIII)
in which Z is leaving group in the presence of a base;
(f) for those compounds of formula I in which X is -S-, -SCH₂₋ or -CH₂S- wherein the sulphur atom bears one or two oxygen atoms, oxidising the corresponding compound of formula I in which X is -S-, -SCH₂₋ or -CH₂S-;
and whereafter when a pharmaceutically acceptable salt is required treating the compound of formula I with an acid which affords a physiologicaly acceptable anion or a base which affords a physiologically acceptable cation.

10. A pharmaceutical composition comprising a compound of formula I, or a pharmaceutically acceptable salt thereof thereof, as claimed in any one of claims 1 to 8 together with a pharmaceutcially acceptable diluent or carrier.

11. The use of a compound of formula I, or a pharmaceutically acceptable salt thereof, as claimed in any one of claims 1 to 8 for the manufacture of a medicament for inhibiting cholesterol biosynthesis.

12. The use as claimed in claim 11 for the manufacture of a medicament for treating hypercholesterolemia or atherosclerosis.

## Patentansprüche

1. Verbindung der Formel I: 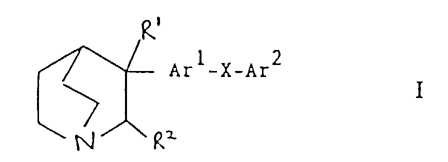 oder ein pharmazeutisch akzeptables Salz davon, worin:
R¹ Hydroxy ist;
R² Wasserstoff ist;
X ausgewählt wird aus -CH₂CH₂-, -CH=CH-, -C≡C-, -CH₂O-, -OCH₂-, -CH₂NH-, -NHCH₂-, -CH₂CO-, -COCH₂-, -CH=N-,-N=CH-, -O-, -NH-, -CO-, -CH₂-, -CH₂S-, -SCH₂- und -S- (worin das Schwefelatom in den letzteren drei Gruppen gegebenenfalls ein oder zwei Sauerstoffatome tragen kann);
Ar¹ eine Phenylen-Einheit ist;
Ar² eine heterocyclische Einheit ist;
und worin eine oder beide der Ar¹- und Ar²-Gruppen gegebenenfalls einen oder mehrere Substituenten tragen können, welche unabhängig ausgewählt werden aus Halogeno, Hydroxy, Amino, Nitro, Cyano, Carboxy, Carbamoyl, (C₁₋₆)-Alkyl, (C₂₋₆)-Alkenyl, (C₂₋₆)-Alkinyl, (C₁₋₆)-Alkoxy, (C₁₋₆)-Alkylamino, Di[(C₁₋₆)-alkyl]amino, N-(C₁₋₆)-Alkylcarbamoyl, Di-N,N-[(C₁₋₆)-alkyl]carbamoyl, (C₁₋₆)-Alkoxycarbonyl, (C₁₋₆)-Alkylthio, (C₁₋₆)-Alkylsulfinyl, (C₁₋₆)-Alkylsulfonyl, Halogeno-(C₁₋₆)-alkyl, (C₁₋₆)-Alkanoylamino, Ureido, N'-(C₁₋₆)-Alkylureido, (C₁₋₆)-Alkanoyl und Oxim-Derivaten davon und O-(C₁₋₆)-Alkylethern dieser Oxim-Derivate.

2. Verbindung gemäß Anspruch 1, worin Ar¹ eine 1,4-Phenylen-Einheit ist.

3. Verbindung gemäß einem der vorhergehenden Ansprüche, worin eine oder beide der Gruppen Ar¹ und Ar² gegebenenfalls unsubstituiert sein können oder einen oder mehrere Substituenten tragen können, welche unabhängig ausgewählt werden aus Halogeno, Hydroxy, Amino, Nitro, (C₁₋₆)-Alkyl, (C₂₋₆)-Alkenyl, (C₁₋₆)-Alkoxy, (C₁₋₆)-Alkanoyl und Oxim-Derivaten davon und O-(C₁₋₆)-Alkylethern dieser Oxime, Halogeno-(C₁₋₆)-alkyl und (C₁₋₆)-Alkanoylamino.

4. Verbindung gemäß einem der vorhergehenden Ansprüche, worin Ar² ausgewählt wird aus Furyl, Pyrrolyl, Thienyl, Pyridyl, Pyrimidinyl, Pyridazinyl, Imidazolyl, Oxazolyl, Isooxazolyl, Thiazolyl, Isothiazolyl, Oxadiazolyl, 1,2,3-Triazolyl, 1,2,4-Triazolyl, Benzfuranyl, Chinolyl, Isochinolyl, Benzimidazolyl, Indolyl, Benzothiazolyl, Benzodioxolyl, Benzodioxanyl und Benzodihydrofuranyl.

5. Verbindung gemäß einem der vorhergehenden Ansprüche, worin X ausgewählt wird aus -CH₂CH₂-, -CH=CH-, -C≡C-,-CH₂O-, -OCH₂-, -CH₂NH-, -NHCH₂-, -CH₂CO-, -COCH₂-, -O-, -NH-, -CH₂S-, -SCH₂-, -S- und wobei das Schwefelatom in den letzteren drei Gruppen gegebenenfalls ein oder zwei Sauerstoffatome tragen kann.

6. Verbindung gemäß einem der vorhergehenden Ansprüche, worin Ar² ausgewählt wird aus 2-Thienyl, 2-Pyridyl, 3-Pyridyl, 2-Pyrrolyl, 2-Imidazolyl, 1,2,3-Triazol-4-yl, 1,2,4-Triazol-3-yl, 3-Chinolyl, 2-Chinolyl, 2-Thiazolyl, 5-Thiazolyl, 1-Isochinolyl, 3-Isochinolyl, 2-Benzimidazolyl, 2-Benzothiazolyl, 5-Oxadiazolyl, 2-Indolyl und 3-Indolyl, welche gegebenenfalls einen oder zwei Substituenten enthalten können, die unabhängig ausgewählt werden aus Fluor, Chlor, Brom, Jod, Hydroxy, Nitro, Methyl, Ethyl, Propyl, Isopropyl, Butyl, Isobutyl, sek-Butyl, Allyl, Methoxy, Ethoxy, Propoxy, Isopropoxy, Butoxy, Formyl, Acetyl, Propionyl, Butyryl und Oxim-Derivaten der letzteren vier Gruppen und O-Methyl-, Ethyl-, Propyl-, Isopropyl- und Butylethern dieser Oxime, Acetamido, Propionamido, Isopropionamido, Fluormethyl, Difluormethyl und Trifluormethyl.

7. Verbindung gemäß einem der vorhergehenden Ansprüche, worin X ausgewählt wird aus -CH=CH-, -CH₂O- und -C≡C-.

8. Verbindung gemäß Anspruch 1, welche ausgewählt wird aus:
3-(4-[(E)-2-(3-Pyridyl)vinyl]phenyl)chinuclidin-3-ol;
3-(4-[(Z)-2-(3-Pyridyl)vinyl]phenyl)chinuclidin-3-ol;
und ihren pharmazeutisch akzeptablen Salzen.

9. Verfahren zur Herstellung einer Verbindung der Formel I oder eines pharmazeutisch akzeptablen Salzes davon gemäß Anspruch 1, wobei das Verfahren ausgewählt wird aus:
(a) für Verbindungen, in denen R¹ Hydroxy ist und R² Wasserstoff ist, Umsetzung eines metallorganischen Derivats der Formel III:
Ar²-X-Ar¹-M (III)
worin M ein Metallatom oder ein Derivat davon ist, mit Chinuclidin-3-on;
(b) für solche Verbindungen der Formel I, worin X -CH=CH- ist, Umsetzung einer Verbindung der Formel IV: 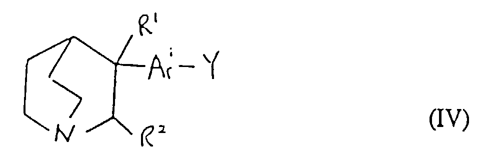 worin Y -CHO ist, mit einer Verbindung der Formel V, worin W ein Halogenatom (wie z.B. Chlor) ist, in Gegenwart einer Base;
(c) für Verbindungen der Formel I, worin X -C≡C- ist, Umsetzung einer Verbindung der Formel IV: 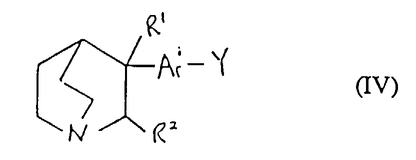 worin Y eine Abgangsgruppe ist, mit einer Verbindung der Formel VI:
Ar²-C≡CM (VI)
worin M ein Metallatom ist;
(d) für Verbindungen der Formel I, worin X -OCH₂-, -NHCH₂- oder -SCH₂- ist, Umsetzung einer Verbindung der Formel IV: 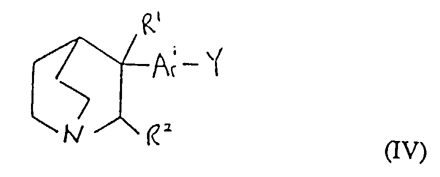 worin Y -OH, -NH₂ oder -SH ist, mit einer Verbindung der Formel VII:
Z-CH₂Ar² (VII)
worin Z eine Abgangsgruppe ist, in Gegenwart einer Base;
(e) für Verbindungen der Formel I, worin X -CH₂O-, -CH₂NH- oder -CH₂S- ist, durch Reaktion einer Verbindung der Formel IV: 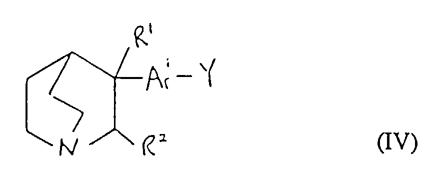 worin Y -CH₂OH, -CH₂NH₂ oder -CH₂SH ist, mit einer Verbindung der Formel VIII:
Z-Ar² (VIII)
worin Z eine Abgangsgruppe ist, in Gegenwart einer Base;
(f) für Verbindungen der Formel I, worin X -S-, -SCH₂- oder -CH₂S- ist und das Schwefelatom 1 oder 2 Sauerstoffatome trägt, Oxidation der entsprechenden Verbindung der Formel I, worin X -S-, -SCH₂- oder -CH₂S- ist;
und danach, wenn ein pharmazeutisch akzeptables Salz gewünscht wird, Behandlung der Verbindung der Formel I mit einer Säure, die ein physiologisch akzeptables Anion liefert, oder einer Base, die ein physiologisch akzeptables Kation liefert.

10. Pharmazeutische Zusammensetzung, umfassend eine Verbindung der Formel I oder ein pharmazeutisch akzeptables Salz davon gemäß einem der Ansprüche 1 bis 8 zusammen mit einem pharmazeutisch akzeptablen Verdünnungsmittel oder Träger.

11. Verwendung einer Verbindung der Formel I oder eines pharmazeutisch akzeptablen Salzes davon gemäß einem der Ansprüche 1 bis 8 zur Herstellung eines Medikaments zur Inhibierung der Cholesterin-Biosynthese.

12. Verwendung gemäß Anspruch 11 zur Herstellung eines Medikaments zur Behandlung der Hypercholesterinämie oder Atherosklerose.

## Revendications

1. Composé de formule I, 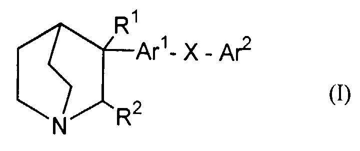 ou un sel acceptable pharmaceutiquement de celui-ci, dans laquelle:
R¹ est un hydroxy;
R² est un hydrogène;
X est choisi parmi -CH₂CH₂-, -CH= CH-, -C≡C-, -CH₂O-, -OCH₂-, -CH₂NH-, -NHCH₂-, -CH₂CO-, -COCH₂-, -CH=N-, -N=CH-, -O-, -NH-, -CO-, -CH₂-, - CH₂S-, -SCH₂- and -S- (l'atome de soufre dans les trois derniers groupes pouvant éventuellement porter un ou deux atomes d'oxygène);
Ar¹ est un segment phénylène;
Ar² est un segment hétérocyclique;
et dans laquelle un ou les deux Ar¹ and Ar² peuvent éventuellement porter un ou plusieurs substituants indépendamment choisis parmi un halogéno, hydroxy, amino, nitro, cyano, carboxy, carbamoyle, alkyle(en C1-6), alcényle(en C2-6), alcynyle(en C2-6), alcoxy (en C1-6), alkylamino (en C1-6), di-[alkyl (en C1-6)amino]N-alkyl (en C1-6)carbamoyle, di-N,N-[alkyl(en C1-6)]carbamoyle, alcoxycarbonyle (en C1-6), alkylthio (en C1-6), alkylsulfinyle(en C1-6), alkylsulfonyle (en C1-6), halogéno alkyle(en C1-6), alkanoylamino(en C1-6), uréido, N'-alkyl (en C1-6)uréido, alcanoyle (en C1-6) et les dérivés oximes de ceux-ci et les éthers O- alkyle (en C1-6) desdits dérivés oximes.

2. Composé selon la revendication 1, caractérisé en ce que Ar¹ est un segment 1,4-phénylène.

3. Composé selon l'une quelconque des revendications précédentes, caractérisé en ce que l'un ou les deux Ar¹ and Ar² peuvent éventuellement être non substitués ou peuvent porter un ou plusieurs substituants choisis indépendamment parmi un halogéno, hydroxy, amino, nitro, alkyle (en C1-6), alcényle(en C2-6), alcoxy.(en C1-6), alcanoyle (en C1-6)et les dérivés oxime de celui-ci et les éthers O-alkyle (en C1-6) desdits oximes, halogénoalkyle (en C1-6) et alcanoylamino(en C1-6).

4. Composé selon l'une quelconque des revendications précédentes, caractérisé en ce que Ar² est choisi parmi un furyle, pyrrolyle, thiényle, pyridyle, pyrimidinyle, pyridazinyle, imidazolyle, oxazolyle, isooxazolyle, thiazolyle, isothiazolyle, oxadiazolyle, 1,2,3-triazolyle, 1,2,4-triazolyle, benzofuranyle, quinolyle, isoquinolyle, benzimidazolyle, indolyle, benzothiazolyle, benzodioxolyle, benzodioxanyle et benzodihydrofuranyle.

5. Composé selon l'une quelconque des revendications précédentes, caractérisé en ce que X est choisi parmi -CH₂CH₂-, -CH= CH-, -C≡C-, - CH₂O-, -OCH₂-, -CH₂NH-, -NHCH₂-, -CH₂CO-, -COCH₂-, -O-, -NH-, - CH₂S-, -SCH₂-, -S- et l'atome de soufre dans les trois derniers groupes pouvant porter éventuellement un ou deux atomes d'oxygène.

6. Composé selon l'une quelconque des revendications précédentes, caractérisé en ce que Ar² est choisi parmi un 2-thiényle, 2-pyridyle, 3-pyridyle, 2-pyrrolyle, 2-imidazolyle, 1,2,3-triazol-4-yle, 1,2,4-triazol-3-yle, 3-quinolyle, 2-quinolyle, 2-thiazolyle, 5-thiazolyle, 1-isoquinolyle, 3-isoquinolyle, 2-benzimidazolyle, 2-benzothiazolyle, 5-oxadiazoyle, 2-indolyle et 3-indolyle, qui peut éventuellement porter un ou deux substituants choisis indépendamment parmi un fluoro, chloro, bromo, iodo, hydroxy, nitro, méthyle, éthyle, propyle, isopropyle, butyle, isobutyle, sec-butyle, allyle, méthoxy, éthoxy, propoxy, isopropoxy, butoxy, formyle, acétyle, propionyle, butyryle et les dérivés oximes des quatre derniers groupes et les éthers O-méthyle, éthyle, propyle, isopropyle et butyle desdits oximes, acétamido, propionamido, isopropionamido, fluorométhyle difluorométhyle et trifluorométhyle.

7. Composé selon l'une quelconque des revendications précédentes, caractérisé en ce que X est choisi parmi -CH= CH-, -CH₂O- et -C≡C-.

8. Composé selon la revendication 1, caractérisé en ce qu'il est choisi parmi:
le 3-(4-[(E)-2-(3-pyridyl)vinyl]phényl)quinuclidin-3-ol ;
le 3-(4-[(Z)-2-(3-pyridyl)vinyl]phényl)q uinuclidin-3-ol;
et leurs sels acceptables pharmaceutiquement.

9. Procédé de préparation d'un composé de formule 1, ou un sel acceptable pharmaceutiquement de celui-ci, selon la revendication 1, le procédé étant choisi parmi:
(a) pour ceux des composés dans lesquels R¹ est l'hydroxy et R² est l'hydrogène, la réaction d'un dérivé organométallique de formule III:
Ar²-X-Ar¹-M (III)
dans laquelle M est un atome de métal ou un dérivé de celui-ci, avec la quinuclidin-3-one;
(b) pour ceux des composés de formule I dans laquelle X est -CH=CH-, la réaction d'un composé de formule IV: 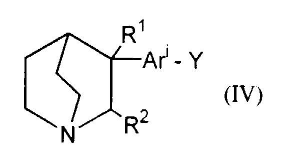 dans laquelle Y est -CHO avec un composé de formule V:
Ar²-CH₂P⁺Ph₃W⁻ (V)
dans laquelle W est un atome d'halogène (tel qu'un chloro), en présence d'une base;
(c) pour ceux des composés de formule I dans laquelle X est -C≡C-, la réaction d'un composé de formule IV: 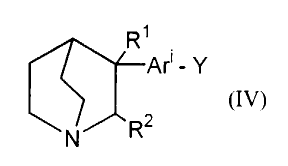 dans laquelle Y est un groupe se séparant avec un composé de formule VI:
Ar²-C≡CM (VI)
dans laquelle M est un atome de métal;
(d) pour ceux des composés de formule I dans laquelle X est -OCH₂-, -NHCH₂- ou -SCH₂-, la réaction d'un composé de formule IV: 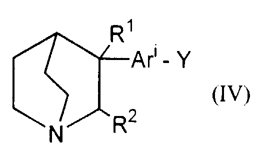 dans laquelle Y est -OH, -NH₂ ou -SH, avec un composé de formule VII:
Z- CH₂Ar² (VII)
dans laquelle Z est un groupe se séparant, en présence d'un base;
(e) pour ceux des composés de formule I dans laquelle X est -CH₂O-, -CH₂NH ou -CH₂S- par réaction d'un composé de formule IV: 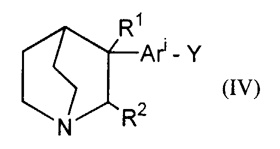 dans laquelle Y est -CH₂OH, -CH₂NH₂ ou -CH₂SH avec un composé de formule VIII:
Z- Ar² (VIII)
dans laquelle Z est un groupe se séparant, en présence d'une base;
(f) pour ceux des composés de formule I dans laquelle X est -S-, -SCH₂- ou -CH₂S-, l'atome de soufre portant un ou deux atomes d'oxygène, l'oxydation du composé correspondant de formule I dans laquelle X est -S-, -SCH₂- pu -CH₂S-;
et ensuite quand un sel acceptable pharmaceutiquement est requis, le traitement du composé de formule I avec un acide qui fournit un anion acceptable physiologiquement ou une base qui fournit un cation acceptable physiologiquement.

10. Composition pharmaceutique comprenant un composé de formule I, ou un sel acceptable pharmaceutiquement de celui-ci, selon l'une quelconque des revendications 1 à 8, avec un diluant ou un support acceptable pharmaceutiquement.

11. Utilisation d'un composé de formule I, ou un sel acceptable pharmaceutiquement de celui-ci, selon l'une quelconque des revendications 1 à 8, pour la fabrication d'un médicament pour l'inhibition de la biosynthèse du cholestérol.

12. Utilisation selon la revendication 11 pour la fabrication d'un médicament pour le traitement de l'hypercholestérolémie et l'artériosclérose.
